# EUROPEAN PATENT APPLICATION

(11) **EP 2 405 000 A1**
(43) Date of publication of application: **11.01.2012**
(21) Application number: 10168636.8
(22) Date of filing: 06.07.2010
(51) Int. Cl.: C12N 15/10, C40B 40/06, C40B 40/08

(54) **Synthesis of chemical libraries**

(71) Applicant: Alacris Theranostics GmbH, 68163 Mannheim (DE)
(72) Inventor: Glöckler Jörn, 10783 Berlin (DE); Lehrach Hans, 14129 Berlin (DE)
(74) Representative: Vossius & Partner

(57) **Abstract**

The present invention pertains to a method for synthesizing a combinatorial library comprising the following steps: (i) providing a library of template nucleic acids that are partially double-stranded, wherein each template nucleic acid comprises a reactive group, and wherein the two strands in the double-stranded region are covalently linked, (ii) contacting said template nucleic acids with a library of adapter oligonucleotides, wherein each adapter oligonucleotide of the library comprises a first sequence of 2 or more defined nucleotide residues at one end, and a second sequence of from 7 to 60, nucleotide residues, (iii) ligating those of the adapter oligonucleotides to the last nucleotide of the double-stranded portion of the template nucleic acid which have hybridized with their first sequence to the single stranded portion of the template nucleic acid in immediate proximity to the double-stranded portion of the template nucleic acid, (iv) sorting said template nucleic acids according to the second sequences of the adapter oligonucleotides, (v) chemically modifying said reactive group of the template nucleic acids, (vi) removing the second sequence of the adapter oligonucleotide by cleavage of the ligated adapter oligonucleotides between the first sequence and the second sequence or by enzymatic digestion with an exonuclease, and (vii) repeating steps (ii) to (vi) as required.

SEQUENCE LISTING

<110>  Max-Planck-Gesellschaft zur Förderung der Wissenschaften
       e.V.

<120>  Synthesis of chemical libraries

<130>  S1784 EP BLN

<160>  36

<170>  PatentIn version 3.5

<210>  1
<211>  27
<212>  DNA
<213>  Artificial Sequence

<220>
<223>  primer/probe


<220>
<221>  misc_feature
<222>  (4)..(4)
<223>  abasic site

<220>
<221>  misc_feature
<222>  (5)..(7)
<223>  n is a, c, g, t or u

<400>  1
aagnnnncgg tggaagtata tgtacct                                          27


<210>  2
<211>  26
<212>  DNA
<213>  Artificial Sequence

<220>
<223>  primer/probe


<220>
<221>  misc_feature
<222>  (4)..(4)
<223>  abasic site

<220>
<221>  misc_feature
<222>  (5)..(6)
<223>  n is a, c, g, t or u

<400>  2
acgnnngatg tgaagtgtgt atccac                                           26


<210>  3
<211>  27
<212>  DNA
<213>  Artificial Sequence

<220>
<223>  primer/probe

<220>
<221> misc_feature
<222> (4)..(4)
<223> abasic site

<220>
<221> misc_feature
<222> (5)..(7)
<223> n is a, c, g, t or u

<400> 3
aggnnnngag cgtgtaatcc gatctaa 27


<210> 4
<211> 27
<212> DNA
<213> Artificial Sequence

<220>
<223> primer/probe


<220>
<221> misc_feature
<222> (4)..(4)
<223> abasic site

<220>
<221> misc_feature
<222> (5)..(7)
<223> n is a, c, g, t or u

<400> 4
atgnnnngcc gtgttacgac ccgttaa 27


<210> 5
<211> 27
<212> DNA
<213> Artificial Sequence

<220>
<223> primer/probe


<220>
<221> misc_feature
<222> (4)..(4)
<223> abasic site

<220>
<221> misc_feature
<222> (5)..(7)
<223> n is a, c, g, t or u

<400> 5
cagnnnngca ctatatgcac agctcga 27


<210> 6
<211> 27
<212> DNA
<213> Artificial Sequence

<220>
<223> primer/probe

<220>
<221>  misc_feature
<222>  (4)..(4)
<223>  abasic site

<220>
<221>  misc_feature
<222>  (5)..(7)
<223>  n is a, c, g, t or u

<400>  6
ccgnnnncgg ataaagacac agttcgc                                          27


<210>  7
<211>  27
<212>  DNA
<213>  Artificial Sequence

<220>
<223>  primer/probe


<220>
<221>  misc_feature
<222>  (4)..(4)
<223>  abasic site

<220>
<221>  misc_feature
<222>  (5)..(7)
<223>  n is a, c, g, t or u

<400>  7
cggnnnngct tcattactac tatgcgc                                          27


<210>  8
<211>  27
<212>  DNA
<213>  Artificial Sequence

<220>
<223>  primer/probe


<220>
<221>  misc_feature
<222>  (4)..(4)
<223>  abasic site

<220>
<221>  misc_feature
<222>  (5)..(7)
<223>  n is a, c, g, t or u

<400>  8
ctgnnnncgc gtaactttat ctaaggc                                          27


<210>  9
<211>  27
<212>  DNA
<213>  Artificial Sequence

<220>
<223>  primer/probe

<220>
<221> misc_feature
<222> (4)..(4)
<223> abasic site

<220>
<221> misc_feature
<222> (5)..(7)
<223> n is a, c, g, t or u

<400> 9
gagnnnncga catataagta tcagccg 27


<210> 10
<211> 27
<212> DNA
<213> Artificial Sequence

<220>
<223> primer/probe


<220>
<221> misc_feature
<222> (4)..(4)
<223> abasic site

<220>
<221> misc_feature
<222> (5)..(7)
<223> n is a, c, g, t or u

<400> 10
gcgnnnncga caatacttga cagcacg 27


<210> 11
<211> 27
<212> DNA
<213> Artificial Sequence

<220>
<223> primer/probe


<220>
<221> misc_feature
<222> (4)..(4)
<223> abasic site

<220>
<221> misc_feature
<222> (5)..(7)
<223> n is a, c, g, t or u

<400> 11
gggnnnncga agcgtatgga agctacc 27


<210> 12
<211> 27
<212> DNA
<213> Artificial Sequence

<220>

<223>  primer/probe

<220>
<221>  misc_feature
<222>  (4)..(4)
<223>  abasic site

<220>
<221>  misc_feature
<222>  (5)..(7)
<223>  n is a, c, g, t or u

<400>  12
gtgnnnnctg agaatatgag agacgcc                                          27

<210>  13
<211>  27
<212>  DNA
<213>  Artificial Sequence

<220>
<223>  primer/probe

<220>
<221>  misc_feature
<222>  (4)..(4)
<223>  abasic site

<220>
<221>  misc_feature
<222>  (5)..(7)
<223>  n is a, c, g, t or u

<400>  13
tagnnnnctc actaagttca tagaccg                                          27

<210>  14
<211>  27
<212>  DNA
<213>  Artificial Sequence

<220>
<223>  primer/probe

<220>
<221>  misc_feature
<222>  (4)..(4)
<223>  abasic site

<220>
<221>  misc_feature
<222>  (5)..(7)
<223>  n is a, c, g, t or u

<400>  14
tcgnnnncca gaaggatata cacgcgc                                          27

<210>  15
<211>  27
<212>  DNA
<213>  Artificial Sequence

<220>
<223>  primer/probe


<220>
<221>  misc_feature
<222>  (4)..(4)
<223>  abasic site

<220>
<221>  misc_feature
<222>  (5)..(7)
<223>  n is a, c, g, t or u

<400>  15
tggnnnncct aagaccgtag aatagcc                                          27


<210>  16
<211>  27
<212>  DNA
<213>  Artificial Sequence

<220>
<223>  primer/probe


<220>
<221>  misc_feature
<222>  (4)..(4)
<223>  abasic site

<220>
<221>  misc_feature
<222>  (5)..(7)
<223>  n is a, c, g, t or u

<400>  16
ttgnnnnacc tgcgtaaagg atagcac                                          27


<210>  17
<211>  20
<212>  DNA
<213>  Artificial Sequence

<220>
<223>  probe

<400>  17
aggtacatat acttccaccg                                                  20


<210>  18
<211>  20
<212>  DNA
<213>  Artificial Sequence

<220>
<223>  probe

<400>  18
gtggatacac acttcacatc                                                  20


<210>  19
<211>  20
<212>  DNA

<213>  Artificial Sequence

<220>
<223>  probe

<400>  19
ttagatcgga ttacacgctc                                                    20


<210>  20
<211>  20
<212>  DNA
<213>  Artificial Sequence

<220>
<223>  probe

<400>  20
ttaacgggtc gtaacacggc                                                    20


<210>  21
<211>  20
<212>  DNA
<213>  Artificial Sequence

<220>
<223>  probe

<400>  21
tcgagctgtg catatagtgc                                                    20


<210>  22
<211>  20
<212>  DNA
<213>  Artificial Sequence

<220>
<223>  probe

<400>  22
gcgaactgtg tctttatccg                                                    20


<210>  23
<211>  20
<212>  DNA
<213>  Artificial Sequence

<220>
<223>  probe

<400>  23
gcgcatagta gtaatgaagc                                                    20


<210>  24
<211>  20
<212>  DNA
<213>  Artificial Sequence

<220>
<223>  probe

<400>  24
gccttagata aagttacgcg                                                    20

<210>  25
<211>  20
<212>  DNA
<213>  Artificial Sequence

<220>
<223>  probe

<400>  25
cggctgatac ttatatgtcg                                                    20


<210>  26
<211>  20
<212>  DNA
<213>  Artificial Sequence

<220>
<223>  probe

<400>  26
cgtgctgtca agtattgtcg                                                    20


<210>  27
<211>  20
<212>  DNA
<213>  Artificial Sequence

<220>
<223>  probe

<400>  27
ggtagcttcc atacgcttcg                                                    20


<210>  28
<211>  20
<212>  DNA
<213>  Artificial Sequence

<220>
<223>  probe

<400>  28
ggcgtctctc atattctcag                                                    20


<210>  29
<211>  20
<212>  DNA
<213>  Artificial Sequence

<220>
<223>  probe

<400>  29
cggtctatga acttagtgag                                                    20


<210>  30
<211>  20
<212>  DNA
<213>  Artificial Sequence

<220>
<223>  probe

<400>  30
gcgcgtgtat atccttctgg                                                    20


<210>  31
<211>  20
<212>  DNA
<213>  Artificial Sequence

<220>
<223>  probe

<400>  31
ggctattcta cggtcttagg                                                    20


<210>  32
<211>  20
<212>  DNA
<213>  Artificial Sequence

<220>
<223>  probe

<400>  32
gtgctatcct ttacgcaggt                                                    20


<210>  33
<211>  54
<212>  DNA
<213>  Artificial Sequence

<220>
<223>  primer


<220>
<221>  misc_feature
<222>  (20)..(21)
<223>  n is a, c, g, or t

<220>
<221>  misc_feature
<222>  (23)..(24)
<223>  n is a, c, g, or t

<220>
<221>  misc_feature
<222>  (26)..(27)
<223>  n is a, c, g, or t

<220>
<221>  misc_feature
<222>  (29)..(30)
<223>  n is a, c, g, or t

<220>
<221>  misc_feature
<222>  (32)..(33)
<223>  n is a, c, g, or t

<220>
<221>  misc_feature
<222>  (35)..(36)
<223>  n is a, c, g, or t

<400>  33
tgacaccgta cctgctctcn ncnncnncnn cnncnnaacc acgccaggga ctat                54

<210>  34
<211>  37
<212>  DNA
<213>  Artificial Sequence

<220>
<223>  primer/probe

<400>  34
gacgcgattt tttcgcgtca tagtccctgg cgtgctt                                   37

<210>  35
<211>  18
<212>  DNA
<213>  Artificial Sequence

<220>
<223>  primer/probe

<400>  35
tgacaccgta cctgctct                                                        18

<210>  36
<211>  18
<212>  DNA
<213>  Artificial Sequence

<220>
<223>  primer

<400>  36
atagtccctg gcgtgctt                                                        18

## Description

### Field of the invention

This invention is in the field of biology, chemistry, and molecular biology, more in particular in the field of nucleic acid encoded chemical libraries.

### Background of the invention

The high-throughput screening, selection and *in vitro* evolution of functional compounds such as ligands to biological targets is a very important aspect of drug design. Traditionally, large libraries of chemical compounds are screened for a desired property, e.g. binding or inhibition of a particular protein that is known to be involved in a certain disease. Such libraries may for example be designed using cheminformatics and the compounds may be synthesised using combinatorial organic chemistry, e.g. Click chemistry (Kolb et al. (2001), Angew. Chem. Int. Ed. 40(11):2004-2021). Typical libraries used in the pharmaceutical industry comprise hundreds of thousands or even millions of different compounds. Some libraries may also comprise natural compounds. Also biochemical methods such as SELEX (Tuerk & Gold (1990), Science 249:505--5I0; Ellington & Szostak (1990), Nature 345:818-822) or phage display (Smith (1985), Science 228:1315-1317; McCafferty et al. (i990), Nature 348:552-554) are routinely used for screening biological ligands.

More recently, DNA Encoded Chemical Libraries (DEL) have been developed (reviewed e.g. in Scheuermann & Neri (2010), ChemBioChem 11:931-937 or Clark (2010), Curr. Opin. Chem. Biol. 14:396-403). Two general approaches exist: A first in which DNA directs small-molecule synthesis and a second in which nucleic acid is used to record the synthesis of small molecules. A desired feature of such libraries is the link between the phenotype of a certain molecule (i.e. the "cheanotype") to a particular nucleic acid (i.e. the "genotype") which serves as a tag, a "molecular barcode".

Halpin and Harbury (Halpin & Harbury (2004), 1'LoS Biology 2(7):1022-1030) provided a method for directed synthesis of combinatorial chemistry libraries with DNA genes. This method is based on multiple "split-and-pool" steps during which a DNA is read-out and translated into a unique small-molecule structure.

### Summary of the invention

The present invention provides an improved method for synthesizing nucleic acid-encoded combinatorial libraries. As a result of the method of the invention each specific compound in the library is covalently bound to a specific nucleic acid encoding the synthesis of said specific compound.

The present invention provides in a first aspect a method for synthesizing a combinatorial library comprising the following steps:
(i) providing a library of template nucleic acids that are partially double-stranded, wherein each template nucleic acid comprises a reactive group, and wherein the two strands in the double-stranded region are covalently linked,
(ii) contacting said template nucleic acids with a library of adapter oligonucleotides, wherein each adapter oligonucleotide of the library comprises a first sequence of 2 or more defined nucleotide residues at one end, and a second sequence of from 7 to 60, preferably 15 to 30, nucleotide residues,
(iii) ligating those of the adapter oligonucleotides to the last nucleotide of the double-stranded portion of the template nucleic acid which have hybridized with their first sequence to the single stranded portion of the template nucleic acid in immediate proximity to the double-stranded portion of the template nucleic acid,
(iv) sorting said template nucleic acids according to the second sequences of the adapter oligonucleotides,
(v) chemically modifying said reactive group of the template nucleic acids,
(vi) removing the second sequence of the adapter oligonucleotide by cleavage of the ligated adapter oligonucleotides between the first sequence and the second sequence or by enzymatic digestion with an exonuclease,
(vii) repeating steps (ii) to (vi) as required.

In a further aspect the present invention provides a kit for the synthesis of a combinatorial library comprising in one or more containers:
(i) a library of template nucleic acids that are partially double-stranded, wherein each template nucleic acid comprises a reactive group and wherein the two strands in the double-stranded region are covalently linked;
(ii) a library of adapter oligonucleotides, wherein each adapter oligonucleotide of the library comprises a first sequence of 2 or more defined nucleotide residues at one end, and a second sequence of from 7 to 60, preferably 15 to 30, nucleotide residues;
(iii) optionally a library of capture oligonucleotide probes having sequences hybridizing under stringent conditions to the second sequences of the adapter oligonucleotides;
(iv) optionally reagents for modifying the reactive groups of the template nucleic acids;
(v) optionally one or more aqueous buffer solutions, e.g. a stripping buffer solution to remove probes after hybridisation and detection,
(vi) optionally an endonuclease, preferably Endonuclease IV, and
(vii) optionally a ligase, preferably T4 DNA ligase.
   The invention pertains in another aspect to a method for the selection of compounds for a desired property, comprising the steps of
(I) synthesizing a combinatorial library with a method according to the invention,
(II) determining those members of the library which have the desired property.

### Description of Figures

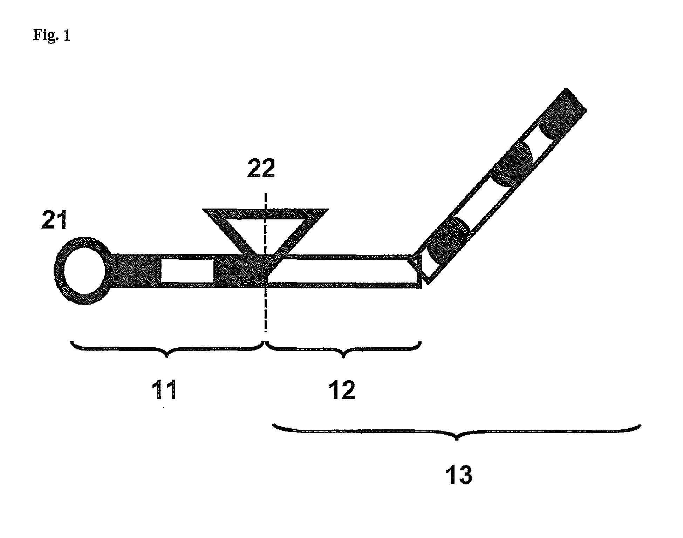

Fig. 1 schematically illustrates a particular embodiment of the adapter oligonucleotide according to the present invention. The adapter oligonucleotide comprises a first sequence (11), and a second sequence (13). The linker sequence (12) is part of the second sequence (13). Further highlighted are the cleavage site (22) between the first sequence (lI) and the linker sequence (12) and a phosphate group (21) at the end of the first sequence (11).

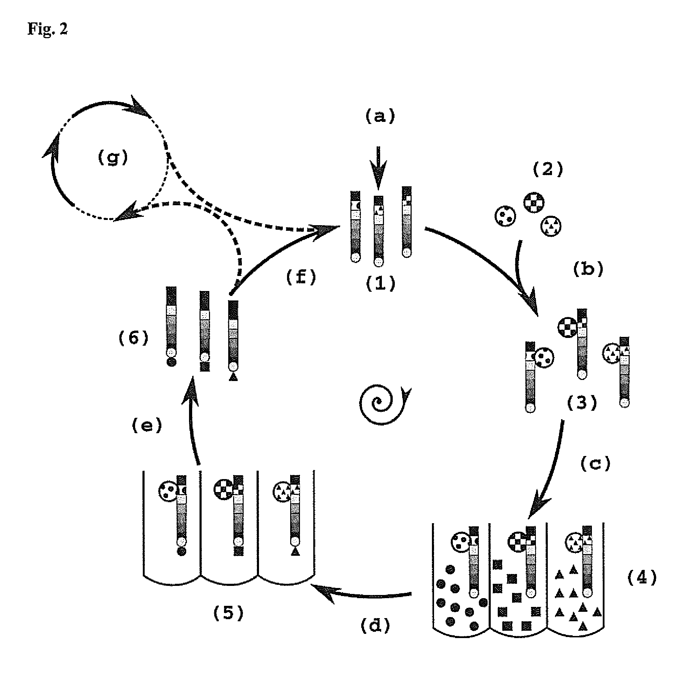

Fig. 2 schematically illustrates a particular embodiment of the method of the invention. Shown is a cycle beginning with the provision (a) of the library (1) of template nucleic acids. In the next step (b) the adapter oligonucleotides (2) are allowed to hybridize to the template nucleic acids; subsequently matching adapter oligonucleotides are ligated to the template nucleic acid (3). In the next step (c) the template nucleic acids are sorted according to the ligated adapter oligonucleotides into defined reaction compartments (4) comprising reaction partners. In the next step (d), the reactive groups of the template nucleic acids can react with reaction partners in the reaction compartments or are otherwise modified yielding modified reactive groups (5). Subsequently in step (e), the template nucleic acids are pooled again and the second sequences of the adapter oligonucleotides are removed. The re-pooled template library (6) can then either directly be subjected to another cycle of nucleic acid encoded synthesis (via route (f)) or it can optionally be subjected to a round of selection (g) for a desired property followed by the generation of a modified template library which in turn then enters the cyclic method.

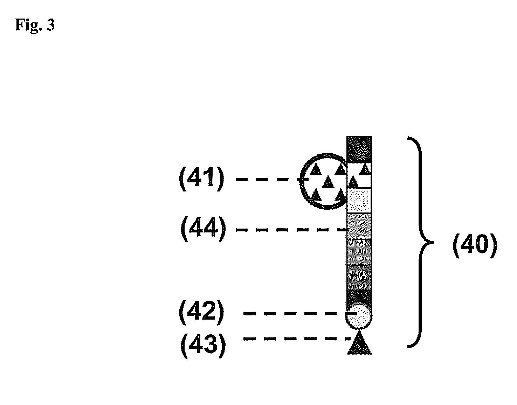

Fig. 3 schematically illustrates a complex of template nucleic acid with adapter oligonucleotide after step (d) in Fig. 2. (40): template nucleic acid with codons and reactive group; (41): adapter oligonucleotide ligated to the template nucleic acid; (42): initial reactive group; (43) modification to reactive group; (44): one of the codons on the template nucleic acid.

### Detailed description of the invention

The present invention provides a new method for synthesizing combinatorial libraries, wherein each compound of the library is covalently linked to a unique nucleic acid, herein designated as "template nucleic acid". The nucleic acid attached to a compound serve as the instruction for synthesis of the compound and as a molecular barcode for identification of the compound. This allows for instance for the selection and directed evolution of compounds by reading out and/or mutating said nucleic acid. In contrast to other methods for nucleic acid encoded library synthesis of the prior art (particularly Halpin & Harbury (2004), PLoS Biology 2(7):1022-1030) the method according to the present invention yields libraries with relatively short nucleic acids and allows for a higher coding density.

Compounds in the context of the present invention can in principle be any compounds that can be chemically and/or enzymatically synthesized from one or more precursor compounds. Hence, the compounds may for example be organic compounds, peptides, polypeptides, nucleic acids or oligosaccharides.

The method of the present invention is based on repeated splitting and pooling of the template nucleic acids. As mentioned above, the template nucleic acids serve as the instruction manual for the synthesis of the compounds of the library. The term "template nucleic acid" may herein be abbreviated as "template". The template nucleic acid comprises consecutive codons of two or more nucleotide residues which each encode single steps of the compound synthesis. Furthermore, the template nucleic acids herein are partially double-stranded and comprise at least one reactive group which subsequently can be chemically modified in reactions steps defined by the sequence of the codons on the template nucleic acid. The double-stranded part of the template nucleic acids is directly adjacent to the coding region which is single-stranded. Initially the template nucleic acids of the library are pooled and contacted with oligonucleotide probes, herein designated "adapter oligonucleotides". Herein, the each adapter oligonucleotide has two sequences: The first sequence which is complementary to the codons on the template and the second sequence which in part may also serve for hybridization to the template but also is used for sorting. The part of the second sequence which also hybridizes to the template and which is adjacent to the first sequence is herein termed "linker sequence". Those of the adapter oligonucleotides that hybridize in proximity to the double-stranded part of the template nucleic acid are ligated to the double-stranded region, thereby extending the double-stranded part. Because the adapter oligonucleotides comprise a part which is not hybridized, the template nucleic acids can be split according to the attached adapter oligonucleotides, e.g. by hybridization to corresponding capture probes. Alternatively, the adapter oligonucleotides may also be directly coupled to e.g. a defined bead. Hence, each template nucleic acid can be transferred to a defined reaction compartment and subjected to a predefined chemical environment, i.e. defined reaction conditions and/or defined reaction partners and/or catalysers such that a chemical or enzymatic reaction which modifies the reactive group on the template nucleic acid might take place. After reaction, the template nucleic acids are pooled again to allow for further cycles of splitting, reacting and pooling. According to the invention, the part of the adapter oligonucleotide that is not complementary to the respective codon on the template nucleic acid is removed between splitting and the next cycle.

The method of the present invention has several advantages over the methods of the prior art. For example, it yields a library of compounds that are each linked to a nucleic acid that encodes the step-by-step synthesis of the attached compound. Furthermore, the attached template nucleic acid can according to the method of the invention be relatively small, since each reaction step only requires one codon, wherein each codon has at least two nucleotide residues. The method according to the invention furthermore allows for an evolution of compounds attached to a template nucleic acid e.g. by using error-prone PCR for amplification of the attached template nucleic acid and subsequent generation of another chemical library.

Hence, the present invention relates in a first aspect to a method for synthesizing a combinatorial library comprising the following steps:
(i) providing a library of template nucleic acids that are partially double-stranded, wherein each template nucleic acid comprises a reactive group, and wherein the two strands in the double-stranded region are covalently linked,
(ii) contacting said template nucleic acids with a library of adapter oligonucleotides, wherein each adapter oligonucleotide of the library comprises a first sequence of 2 or more defined nucleotide residues at one end, and a second sequence of from 7 to 60, preferably 15 to 30, nucleotide residues,
(iii) ligating those of the adapter oligonucleotides to the last nucleotide of the double-stranded portion of the template nucleic acid which have hybridized with their first sequence to the single stranded portion of the template nucleic acid in immediate proximity to the double-stranded portion of the template nucleic acid,
(iv) sorting said template nucleic acids according to the second sequences of the adapter oligonucleotides,
(v) chemically modifying said reactive group of the template nucleic acids,
(vi) removing the second sequence of the adapter oligonucleotide by cleavage of the ligated adapter oligonucleotides between the first sequence and the second sequence or by enzymatic digestion with an exonuclease, and
(vii) repeating steps (ii) to (vi) as required.

The template nucleic acid can for example be a DNA, RNA, LNA (locked nucleic acid), UNA (unlined nucleic acid), PNA (peptide nucleic acid), or a hybrid of any of these. Preferably, however, DNAs are used as template nucleic acids in the context of the present invention. Preferably the length of the template nucleic acid is between 10 and 200 nucleotides or base pairs, most preferably between 15 and 60.

An "oligonucleotide" in the context of the present invention refers to a short nucleic acid polymer, typically with 40 or fewer bases. Although they can in principle be formed by bond cleavage of longer segments, they are typically synthesized by polymerizing individual nucleotide precursors. The nucleotide residues of the oligonucleotides in the context of the present invention may for example be ribonucleotides or desoxyribonucleotides or artificial or modified nucleotides. The adapter oligonucleotides of the invention may preferably be chimeric oligonucleotides, i.e. they may comprise different types of nucleotide residues, e.g. DNA and RNA or DNA, RNA and modified RNA (e.g. LNA), or spiegelmer nucleotides.

The partially double-stranded template nucleic acids of the library may be prepared by different methods, for example by a process comprising the following steps:
(a) providing a library of the single stranded template nucleic acids, wherein each template nucleic acid comprises sequence that is constant within the library,
(b) annealing a primer oligonucleotide to the constant sequence of each of said template nucleic acids, and
(c) ligating or chemically linking said primer oligonucleotide to the template nucleic acid to which it is annealed.

In step (b) of this process of forming the double-stranded template, a primer oligonucleotide is allowed to anneal to the template nucleic acid. Annealing refers to the pairing of the primer oligonucleotide by hydrogen bonds to a complementary sequence on the template nucleic acid, forming a double-stranded polynucleotide. Annealing may be facilitated by decreasing the temperature. The primer oligonucleotide typically has a length of from 10 to 40, preferably 15 to 25 nucleotide residues. In one embodiment, the primer oligonucleotide anneals to either the 3' or the 5' end of the template nucleic acid.
In step (c) of the process of forming the double-stranded template according the annealed primer oligonucleotide is either ligated or chemically linked to the template nucleic acid. In any case a covalent bond between the primer oligonucleotide and the template nucleic acid is formed. When the primer oligonucleotide is annealed to the 3' or the 5' end of the template nucleic acid and the primer oligonucleotide is ligated to the template nucleic acid by a ligase, a hairpin loop can be formed. T4 DNA ligase may for example be used for this ligation.
Covalent linkage of the primer oligonucleotide to the template nucleic acid can be performed by chemical crosslinking, e.g. using a psoralen. Psoralens are furocoumarins, are a class of organic chemical compounds produced by a variety of plants. Psoralens get activated in the presence of UV-A radiation. They form covalent adducts with pyrimidines. Covalent adducts are formed by linking 3, 4 (pyrone) or 4', 5' (furan) edge of psoralen to 5, 6 double bond of thymine. Psoralens can form two types of monoadducts and one diadduct (an interstrand crosslink) reacting with thymine. The crosslinking reaction by psoralens targets TA sequences intercalating in DNA and linking one base of the DNA with the one below it.
In the context of the process of forming the double-stranded template, the ligating in step (c) is preferably performed using a ligase. A preferred ligase herein is T4 DNA ligase. If PNA is used as a template, T4 RNA ligase is preferred. T4 DNA ligase is an enzyme that catalyzes the formation of a phosphodiester bond between juxtaposed 5' phosphate and 3' hydroxyl termini in duplex DNA or RNA. This enzyme will join blunt end and cohesive end termini as well as repair single stranded nicks in duplex DNA, RNA or DNAIRNA hybrids. T4 DNA ligase requires the presence of ATP and Mag2+ in the reaction medium. T4 DNA ligase is commercially available. In one embodiment, a hairpin loop is formed in step (c) of the process of forming the double-stranded template. In such cases ligating in step (c) may be performed using T4 DNA ligase. The primer oligonucleotide may in this embodiment be annealed to either the 3' or the 5' end of the template nucleic acid, preferably to the 3' end. It is particularly preferred that the primer oligonucleotides are annealed to the 3' end of the template nucleic acids, the first sequence is at the 5' end of the adapter oligonucleotides and the reactive group is at the 5' end of the template nucleic acid.

Alternatively, also single-stranded template nucleic acids which form a hairpin loop (also known as stern-loop structure) can be used in the context of the present invention, since they build out double-stranded structure. Typically such nucleic acids comprise sequence stretches that are complementary in nucleotide sequence when read in opposite directions. Hence, in the context of the present invention such nucleic acids are preferred template nucleic acids.

As stated above, the template nucleic acid comprises at least one reactive group which is subsequently modified in the cycles of the method (step (v) as described above). A "reactive group" in the context of the present invention is a functional group that can be modified either chemically or enzymatically under appropriate conditions. Such a reactive group can in the first cycle, i.e. in the initial library of template nucleic acids, for example be an amine group, preferably an amino group, a carboxyl group, or a thiol group, or an isocyanide group, or a ketone group, or an aldehyde group, or a diene group, or a triazole group, or an alkyne group. Preferably the reactive group is at the 3' or 5' end of the template nucleic acid. However, the reactive group may not be at the end of the double stranded region which is to be extended by ligation of the adapter oligonucleotide. Exemplary reactions that might be performed at the reactive groups include Ugi reactions, Diels-Alder cycloadditions, and Click chemistry.
The reactive group may also be an organic compound or a natural product attached to the template nucleic acid. In this case the organic compound or natural product may in the cycles of the method for example be chemically derivatized or modified e.g. by addition, elimination and/or substitution and/or it may be modified by an enzyme. This means that after each cycle of the method, the reactive group may be a different group depending on the modification in the previous cycle. It may for example be that a compound is attached to the template nucleic acid that comprises two or more reactive groups and during each cycle only one of the reactive groups is modified depending on the reaction conditions. In another example, more than one reactive group may be modified in one cycle. By going through multiple cycles of the method of the invention, complex structures such as branched, unbranched, or cyclic polymers can be synthesized. Enzymatic modifications include but are not limited to modifications by a transglutaminase or a protease.

With the method of the invention libraries of, for example, compounds selected from the group consisting of organic compounds, peptides, polypeptides, oligonucleotides, or oligosaccharides, may be synthesized or modified.

As outlined herein above, the template nucleic acid comprises sequences of concatenated codons wherein each codon consist of 2 or more nucleotides, preferably, 2, 3, 4, 5, 6 or 7 nucleotides, more preferably 2, 3 or 4 nucleotides. Each codon ultimately codes for specific reaction parameters or reaction conditions to which the reactive groups are subjected in each cycle of the method. Codons may for example encode the reaction temperatures, solvents, pressure, pH, incubation time, light, catalysers, volume, UV radiation, non-covalent additives, enzymatic modifications, cyclization, branching etc. and/or they may encode the presence of specific reaction partners. From number n of nucleotides in each codon the number of different reaction conditions in one cycle can be calculated by 4ⁿ, i.e. a codon of 3 nucleotide residues results in a maximum of 256 parallel reaction conditions per cycle. The reaction conditions available in one cycle may be changed in another cycle or it may be the same in two or more or even all reaction cycles depending on the desired library. The codons can comprise one or more nucleotides that are constant at a particular position among all codons used, thereby providing a defined frame for the read-out. For example the last, or the first, or the first and the last nucleotide of a codon may be constant.

The codons on the target nucleic acid are complementary to the first sequence on the adapter nucleic acid, i.e. the number of nucleotide residues in a codon corresponds to the number of nucleotide residues in the first sequence of the adapter oligonucleotide. However, since the length of a codon might not be long enough for specific hybridization of the adapter oligonucleotide to the template nucleic acid, the adapter oligonucleotide preferably comprises a sequence of 0 to 10 random or degenerated residues in the second sequence directly adjacent to the first sequence. Said sequence of 0 to 10 random or degenerated residues is herein designated "linker sequence". Together with the first sequence, the linker sequence serves in the method of the present invention for hybridization of the adapter oligonucleotide to the template nucleic acid. In sum the first sequence and the linker sequence have at least 2 nucleotide residues, preferably between 3 and 20 nucleotide residues, more preferably between 5 and 8 nucleotide residues. The second sequence has ― including the linker sequence - preferably from 7 to 60, more preferably 15 to 30 nucleotide residues. In total the adapter oligonucleotide therefore has preferably between 20 and 65 nucleotide residues, more preferably from 20 to 40, most preferably 25 to 30 nucleotide residues. In the method of the present invention the second sequence of the adapter oligonucleotide preferably in part does not hybridize to the template nucleic acid, particularly with the part which is not the linker sequence. This for example allows in subsequent steps of the method for the hybridization of capture oligonucleotide probes to the second sequence. Alternatively, a solid phase, e.g. a bead such as a magnetic bead, may be covalently attached to the second sequence. Such a bead may for example comprise one or more oligonucleotides covalently attached to its surface which allows for sorting. Hence, in a preferred embodiment of the method of the invention the adapter oligonucleotide is attached with the end distal from the first sequence to a solid phase, e.g. a bead.

To cover all possible combinations of codons on the template nucleic acid the library or adapter oligonucleotides must comprise for each codon on the template nucleic acid at least one adapter oligonucleotide which is able to hybridize to the template nucleic acid. The first sequence must sample all possible sequence combinations of the codons used, i.e. 4ⁿ combinations, wherein n is the number of residues per codon. The linker sequence, however, may have a degenerate sequence, i.e. the linker sequence preferably consists of degenerate nucleotides. It may for example comprise ambiguous or universal positions that are able to pair with more than one base positions in the template nucleic acid. For example, the linker sequence may comprise inosine bases which are able to base pair with adenine (A), cytosine (C) and uracil (U) or 3-nitropyrrole or 5-nitroindole which are both universal bases. Alternatively, the library can be designed such that it comprises for each and every sequence on the template nucleic acid an adapter oligonucleotide with complementary first sequences and linker sequences.
The second sequence of the adapter polynucleotides further serves for sorting of ligated adapter oligonucleotides. Every single second sequence must be uniquely assignable to a distinct first sequence and ultimately to the sequence of the respective codon on the template nucleic acid which is to be read-out in a given cycle of the method.

In the context of the method of the present invention, the ligating in step (iii) is preferably performed using a ligase. A preferred ligase herein is T4 DNA ligase.

The splitting of the template nucleic acids takes place in step (iv) of the method. The template nucleic acids are sorted in step (iv) into defined regions on a surface or in a container, e.g. into reaction compartments. Preferably said reaction compartments are on a chip or on a microtiter well plate. The sorting takes place by way of temporary immobilization of the adapter oligonucleotide, which is covalently linked to the template nucleic acid. Immobilization is preferably performed by hybridization of capture oligonucleotides either to the parts of the second sequence of the adapter oligonucleotide that have not hybridized to the template nucleic acid or if applicable to nucleic acids attached to the surface of beads linked to the second sequence. Hence, preferably herein the sorting in step (iv) is by hybridizing an oligonucleotide probe to the second sequence. Consequently, it is preferred herein that the method of the invention comprises the step of contacting the template nucleic acids with capture oligonucleotide probes such that hybridization of the capture oligonucleotide probes to complementary strands of said second sequences of the adapter oligonucleotides may occur. However, also the capture oligonucleotide probes may be attached to solid surfaces, e.g. beads, preferably magnetic beads, in the reaction compartments. In the latter case, the adapter oligonucleotides are preferably not covalently attached to beads or other solid surfaces. In one embodiment of the method according to the invention the capture oligonucleotide probes are attached to the reaction compartments. The direct attachment of the adapter oligonucleotides to a solid surface via its second sequence allows for more stringent washing conditions than by using capture probes that are attached to the solid surface. The capture oligonucleotides herein preferably have a length of from 12 to 25, more preferably around 20 nucleotide residues. Hence, the part of the second sequence to which the capture probes hybridize also have a length of from 12 to 25, more preferably around 20 nucleotide residues.

Once the template nucleic acids are split, e.g. into different reaction compartments, the modification of the reactive group can occur depending on the actual reaction conditions. By repeating the cycle of the method of the present invention multi-step synthesis of compounds can be performed. In each following cycle another modification may take place depending on the reaction conditions provided. In one very particular embodiment of the invention, the synthesis of a library of peptides is encoded by the library of template nucleic acids. In this embodiment, during each cycle of the method the peptide chain attached to the template nucleic acid is extended by one further amino acid residue according to the codon on the template.

Regarding suitable reaction conditions, the method of the present is in principle only limited in the sense that the reaction conditions provided for modifying the reactive group in step (v) must not destroy the template nucleic acid or modify the sequence of the template nucleic acid or cleave the attached compound from the template nucleic acid.

As outlined above, in step (vi) of the method the second sequence is removed after sorting, preferably after the modification of the reactive group. This can either be performed enzymatically, i.e. by using an exonuclease or an endonuclease or chemically. The adapter oligonucleotide is hence designed such that it allows for the removal of the second sequence. Thereby, only the first defined sequence of the adapter oligonucleotide remains. The first defined sequence is the sequence which is complementary to the sequence of the template nucleic acid, i.e. the codon. As mentioned above, removing the second defined sequence including the linker sequence can in principle be achieved in different ways:
a) using an endonuclease that selectively cleaves the adapter oligonucleotide between the first defined sequence and the second sequence,
b) using an exonuclease that stops cleaving before the first defined sequence, or
c) by selective chemical removal of the second sequence.

Chemical removal can for example be achieved by pH dependent cleavage of RNA. This requires the presence of RNA residues in the linker sequence whereas the template nucleic acid and the first sequence must not be RNA in this case. RNA can for instance be cleaved at alkaline pH (e.g. around pH 10 - 12) while DNA is still stable under these conditions while DNA is still stable under these conditions. Hence, in one particular embodiment of the invention cleaving of the adapter oligonucleotide in step (vi) of the method is performed by raising the pH.

In the case an exonuclease, e.g. the Klenow fragment of DNA polymerase I from *E.coli,* is used, the cleavage of the first sequence has to be prevented. This can e.g. be achieved by the use of one or more locked nucleic acid (LNA) residues in the first sequence, particularly at the penultimate position. The ribose moiety of an LNA nucleotide is modified with an extra bridge connecting the 2' oxygen and 4' carbon. The bridge "locks" the ribose in the 3'-endo (North) conformation. Hence, in a particular embodiment the first sequence comprises an LNA nucleotide at the next but one position, i.e. the penultimate position, to the linker sequence.

Different endonucleases may be used for the removal of the second sequence including he linker sequence. One possibility is the use of an RNA endonuclease such as RNase H. This would require that the linker sequence comprise ribonucleotide residues adjacent to the first sequence. Hence, in one particular embodiment of the method the linker sequence is RNA.

Cleaving of the adapter oligonucleotide in step (vi) is in one preferred embodiment of the invention performed by a endonuclease. The most preferred endonuclease to be used in the context of the present invention is Endonuclease IV. Endonuclease IV is an endonuclease that can act on a variety of oxidative damage in DNA. Endonuclease IV enzyme is an apurinic/apyrimidinic endonuclease that will hydrolyse intact abasic sites in DNA. Abasic sites are cleaved at the first phosphodiester bond that is 5' to the abasic site leaving a hydroxyl group at the 3' terminus and a (deoxy)ribose 5'-phosphate at the 5' terminus. Endonuclease IV from different bacterial sources such as *Th. thermophilus* and *E. coli* can be used. Endonuclease IV enzymes are commercially available. Hence, it is preferred herein that the linker sequence of the second sequence of the adapter oligonucleotide comprises at the position proximate to the first sequence an abasic site. As an alternative, the linker sequence may also comprise modified bases at the position proximate to the first sequence such as uracil, urea, oxoguanosine or inosine that can be converted into abasic sites using suitable enzymes such as uracil-DNA glycosylase (UDG) in combination with an exonuclease such as exonuclease VIII in case of uracil bases. Typically such modified bases are recognized by specialized enzymes that are able excise these positions. For the method of the invention it is important that the bond between the last nucleotide of the first sequence and the first nucleotide of the second sequence is cleaved. Depending on the direction of the read-out, phosphorylation at the new free end of the double stranded region, i.e. the last residue of the first sequence may then be required for the next round of the cycle.

The library of template nucleic acids comprises preferably at least 262,144 nucleic acids, more preferably at least 10⁸ nucleic acids.

The library of adapter oligonucleotides preferably comprises at least 16, 64, 256, 1024, or 4096 oligonucleotides.

In step (vii) of the method, i.e. after the modification of the reactive group, the template nucleic acids are preferably again pooled in one compartment.

In principle, the method provided by the present invention works in both reading directions of the template nucleic acid. However, in a particularly preferred embodiment of the invention the template nucleic acid has a free 3' end in the double stranded region and subsequent adapter oligonucleotides are ligated with their 5' end to the 3' end of the respective previous adapter oligonucleotide. This is preferred because most DNA ligases, particularly the preferred T4 DNA ligase, link a free OH group at the 3' end of a first nucleic acid or oligonucleotide to a 5' phosphate group of a second nucleic acid or oligonucleotide.

However, when the method according to the invention is performed in the 3' to 5' direction (on the template strand), an additional step of phosphorylating the 5' end of the elongated template after enzymatic cleavage, particularly with the preferred endonuclease IV, is required in order to allow subsequent ligation in the next cycle. Phosphorylation in these cases can for example be achieved by contacting the 5' end of the adapter oligonucleotide with a kinase and ATP. A suitable kinase is for example Polynucleotide kinase (PNK) which catalyzes the transfer of a phosphate from ATP to the 5' end of either DNA or RNA. Alternatively, the cleavage can be performed with the endonuclease VIII that generates a readily phosphorylated 5' end. The 3' to 5' read-out has a better mismatch detection than the 5' to 3' read-out. The 5' to 3' reading direction is nevertheless preferred because it is less complex than working in the 3' to 5' direction. The directions herein are - unless otherwise stated - the reading directions on the template strand. The direction of extension of the double-stranded portion is vice versa.

In embodiments in which the read-out direction is from 3' to 5' and the second sequence of the adapter oligonucleotide is removed with an exonuclease, it has to be assured that the 5' end of the remaining first sequence is phosphorylated before the next ligation step.

In embodiments in which the read-out direction is from 3' to 5' and the second sequence of the adapter oligonucleotide is removed by endonuclease cleavage at an abasic site, it has to be assured that the 5' end of the remaining first sequence is phosphorylated before the next ligation step. This can for example be achieved by using a suitable endonuclease such as Endonuclease VIII which leaves a phosphorylated 5' end.

The steps of the method of the present invention may require different temperatures such that cycling of the temperature similar as in PCR is required. Particularly the steps in which oligonucleotides or probes are to be annealed or hybridized to another nucleic acid or oligonucleotide may require lowering the temperature to a suitable value which can be calculated by a skilled person according to the length and GC content of the primer probe or oligonucleotide. Hence, for example step (ii) of the method of the present invention may require lowering to temperature to allow annealing of complementary oligonucleotides.

The method according to the present invention may comprise one or more washing steps to wash away non-hybridized oligonucleotides. This particularly applies before sorting the template nucleic acids.

The method of the present invention may be fully or partially automated, e.g. a pipetting roboter and/or a thermocycler may be used. Existing instruments for magnetic particle processing e.g. the Kingfisher Flex (Thermo Fisher Scientific), may be adapted for the method of the present invention.

The present invention also pertains to a combinatorial library obtained or obtainable by the method for synthesizing a combinatorial library as described herein above.

Furthermore, the invention relates to a kit for the synthesis of a combinatorial library comprising in one or more containers:
(i) a library of template nucleic acids that are partially double-stranded, wherein each template nucleic acid comprises a reactive group and wherein the two strands in the double-stranded region are covalently linked;
(ii) a library of adapter oligonucleotides, wherein each adapter oligonucleotide of the library comprises a first sequence of 2 or more defined nucleotide residues at one end, and a second sequence of from 7 to 60, preferably 15 to 30, nucleotide residues;
(iii) optionally a library of capture oligonucleotide probes having sequences hybridizing under stringent conditions to the second sequences of the adapter oligonucleotides;
(iv) optionally reagents for modifying the reactive groups of the template nucleic acids;
(v) optionally one or more aqueous buffer solutions, e.g. a stripping buffer solution to remove probes after hybridisation and detection,
(vi) optionally an endonuclease, preferably Endonuclease IV, and
(vii) optionally a ligase, preferably T4 DNA ligase.

As outlined above, with the method and kit of the present invention libraries of compounds with attached template nucleic acids can be produced. Such libraries can easily comprise more than 10¹⁰ different compounds. Libraries based on codons having n nucleotides per codon and using m codons can comprise up to (4ⁿ)^{m} different compounds; e.g. a library with n=3 and m=5 leads to a maximum of more than 10⁹ different compounds.
The libraries of compounds produced with the method of the present invention can be used for the screening of compounds with desired properties, e.g. a desired activity, affinity, avidity and the like. As outlined above, once promising candidate compounds have been identified during screening, the nucleic acid attached to the candidate compounds may be amplified and sequenced. The sequence reflects the synthesis steps of the identified compounds.

Hence, the invention also relates to a method for the selection of compounds for a desired property, comprising the steps of:
(I) synthesizing a combinatorial library with a method for synthesizing a combinatorial library according to the invention,
(II) determining those members of the library which have the desired property.

The method for the selection of compounds for a desired property may additionally comprise the steps of:
(III) amplifying the template nucleic acids of the members of the library determined in step (II),
(IV) sequencing the template nucleic acids amplified in step (III).

Preferably, the desired property is binding to a target molecule with at least a desired affinity and wherein the method comprises the step of contacting the members of the library with the target molecule.

The compounds of the library synthesized with the method of the invention can be subjected to selection for desired properties *in vivo* or *in vitro.* Suitable methods for determining binding properties for example include immune assays like ELISA, surface plasmon resonance (SPR), gel shift assays, real-time PCR, proximity ligation amplification (PLA) and many more.

After selection the attached template nucleic acid may be synthesized. This can be done by conventional sequencing methods such as Sanger sequencing but also with more recent methods of next generation sequencing such as Pyro sequencing, SOLEXA, SOLID and the like. Some of these methods may require amplification of the template nucleic acid before or during sequencing. Once the template sequence associated with a selected compound is known, the synthesis steps for this compound are also known. This knowledge can for example be used to further refine the synthesis, e.g. by generating new libraries of template nucleic acids and adapter oligonucleotides. It should be noted that the method can also be used for evolution of compounds, e.g. by using error-prone PCR during amplification of the template nucleic acid.

Hence, after an initial synthesis of a chemical library with the method according to the invention, a second, refined chemical library can be generated based on the information obtained from the selection process of the first library. The refined library can for example be generated by providing mutated template nucleic acids and/or by adapting the reaction conditions corresponding to specific codons on the template.

The method for synthesizing a combinatorial library according to the present invention may not only be used for the synthesis of large and/or diverse chemical libraries. It may also be used for the screening for optimal reaction conditions since each codon may encode specific reaction conditions. Hence, the method for synthesizing a combinatorial library according to the present invention can be used for optimizing the synthesis of a certain compound. For example, each codon of one particular cycle could encode specific reaction parameters such as temperature, pH, pressure, solvent and the like. After completion of the steps of the reaction, the yield of the compound to be synthesized can be determined and the sequence of the attached template nucleic acids which resulted in acceptable yields can be determined.

Hence, the present invention also relates to the use of the method for synthesizing a combinatorial library according to the present invention for optimizing the synthesis of a compound, wherein each template nucleic acid encodes for the synthesis of the same compound but under different reaction conditions.

### Examples

### Example 1: Synthesis of a nucleic acid encoded peptide library, selection of peptides binding to Ni-NTA

### Material and methods

The oligonucleotides and template nucleic acids used in this example are given in Tables 1 to 3.

### Enzymes:

- Endonuclease IV, 10 U/µl, BioLabs, New England Biolabs
- T4 DNA ligase, 268 U/µl, Roboklon, Berlin, Germany
- Magnetic beads, Agowa genomic, Berlin, Germany
- Carboxyl magnetic beads (0 0.5 Jim, 600 µmol COOH/g) obtained from Chemicell, Berlin, Germany

### Bead coupling of capture oligonucleotide:

10 mg of carboxyl magnetic particles are washed 2x with 1 ml MES buffer, pH 5. Separate by using the magnetic separator. After second wash step resuspend the particles in 0.25 ml MES buffer containing 10 mg EDC, mix on shaker for 10 min at room temperature. Add 46.92 ug amine group containing capture oligonucleotide to the particles. Mix on a shaker for 2 h at room temperature. After the incubation wash the particles 3x with 1 ml storage buffer (1xPBS, 0.1% Tween, 10 mM Ethanolamine) or (Ix PBS, 0.1% Tween, 10 mM Ethanolamine)

### Hairpin library preparation

ProLib6x2N is initially annealed with the hairpin oligonucleotide P04hpPro3 and ligated with T4 DNA ligase to yield hProLib6x2N before the codon ligation and synthesis as described below.

**Table 1: Adapter oligonucleotides used in Example 1**

| **ID** | **amino acid** | **SEQ ID NO.** | **sequence (5'→ 3')** |
|---|---|---|---|
| aAAG | phenylalanine | SEQ ID NO. 1 | PO₄-AAG-Φ-NNNCGGTGGAAGTATATGTACCT |
| aACG | threonine | SEQ ID NO. 2 | PO₄-ACG-Φ-NNNGATGTGAAGTGTGTATCCAC |
| aAGG | asparagine | SEQ ID NO. 3 | PO₄-AGG-Φ-NNNGAGCGTGTAATCCGATCTAA |
| aATG | methionine | SEQ ID NO. 4 | PO₄-ATG-Φ-NNNGCCGTGTTACGACCCGTTAA |
| aCAG | glutamine | SEQ ID NO. 5 | PO₄-CAG-Φ-NNNGCACTATATGCACAGCTCGA |
| aCCG | proline | SEQ ID ND. 6 | PO₄-CCG-Φ-NNNCGGATAAAGACACAGTTCGC |
| aCGG | arginine | SEQ ID NO. 7 | PO₄-CGG-Φ-NNNGCTTCATTACTACTATGCGC |
| aCTG | isoleucine | SEQ ID NO. 8 | PO₄-CTG-Φ-NNNCGCGTAACTTTATCTAAGGC |
| aGAG | histidine | SEQ ID NO. 9 | PO₄-GAG-Φ-NNNCGACATATAAGTATCAGCCG |
| aGCG | alanine | SEQ ID NO. 10 | PO₄-GCG-Φ-NNNCGACAATACTTGACAGCACG |
| aGGG | glycine | SEQ ID NO. 11 | PO₄-GGG-Φ-NNNCGAAGCGTATGGAAGCTACC |
| aGTG | valine | SEQ ID NO. 12 | PO₄-GTG-Φ-NNNCTGAGAATATGAGAGACGCC |
| aTAG | tyrosine | SEQ ID NO. 13 | PO₄-TAG-Φ-NNNCTCACTAAGTTCATAGACCG |
| aTCG | serine | SEQ ID NO. 14 | PO₄-TCG-Φ-NNNCCAGAAGGATATACACGCGC |
| aTGG | tryptophan | SEQ ID NO. 15 | PO₄-TGG-Φ-NNNCCTAAGACCGTAGAATAGCC |
| aTTG | leucine | SEQ ID NO. 16 | PO₄-TTG-Φ-NNNACCTGCGTAAAGGATAGCAC |

| | | | |
|---|---|---|---|
| Φ: abasic site; PO₄: 5' phosphate | | | |

| | | |
|---|---|---|
| Codon ligation program: | 94°C | 3 min |
| | 37°C | 5 min |
| | 22°C | 30 min |
| | 80°C | 10 min |

The ligation reaction in solution contains 25 pmol of template nucleic acid (hProLib6x2N), 250 pmol of 16 different adapter oligonucleotides with an abasic site (aAAG-aTTG), 335 Units of T4 DNA ligase in 10x T4 buffer in a final volume of 50µl. The mix is incubated at 22°C for 30 min. The T4 DNA ligase is inactivated by incubation at 80°C for 10 min.

**Table 2: Capture oligonucleotides used in Example 1**

| **ID** | **amino acid** | | **sequence (5' → 3')** |
|---|---|---|---|
| hybAAG | phenylalanine | SEQ ID NO. 17 | H₂N - AGGTACATATACTTCCACCG |
| hybACG | threonine | SEQ ID NO. 18 | H₂N - GTGGATACACACTTCACATC |
| hybAGG | asparagine | SEQ ID NO. 19 | H₂N - TTAGATCGGATTACACGCTC |
| hybCTG | methionine | SEQ ID NO. 20 | H₂N - TTAACGGGTCGTAACACGGC |
| hybCAG | glutamine | SEQ ID NO. 21 | H₂N - TCGAGCTGTGCATATAGTGC |
| hybCCG | proline | SEQ ID NO. 22 | H₂N - GCGAACTGTGTCTTTATCCG |
| hybCGG | arginine | SEQ ID NO. 23 | H₂N - GCGCATAGTAGTAATGAAGC |
| hybCTG | isoleucine | SEQ ID NO. 24 | H₂N - GCCTTAGATAAAGTTACGCG |
| hybGAG | histidine | SEQ ID NO. 25 | H₂N - CGGCTGATACTTATATGTCG |
| hybGCG | alanine | SEQ ID NO. 26 | H₂N - CGTGCTGTCAAGTATTGTCG |
| hybGGG | glycine | SEQ ID NO. 27 | H₂N - GGTAGCTTCCATACGCTTCG |
| hybGTG | valine | SEQ ID NO. 28 | H₂N - GGCGTCTCTCATATTCTCAG |
| hybTAG | tyrosine | SEQ ID NO. 29 | H₂N - CGGTCTATGAACTTAGTGAG |
| hybTCG | seine | SEQ ID NO. 30 | H₂N - GCGCGTGTATATCCTTCTGG |
| hybTGG | tryptophan | SEQ ID NO. 31 | H₂N - GGCTATTCTACGGTCTTAGG |
| hybTTG | leucine | SEQ ID NO. 32 | H₂N - GTGCTATCCTTTACGCAGGT |

| | | | |
|---|---|---|---|
| H₂N: 5' amino group | | | |

**Table 3: Templates, hairpin oliogonucleotide and primers used in Example 1**

| **ID** | **function** | **sequence** |
|---|---|---|
| ProLib6x2N SEQ IDNO. 33 | template library | CO₂ - TGACACCGTACCTGCTCTCNNCNNCNNCNNCNNCNNAACCACGCCAGGGACTAT |
| PO4hpPro3 SEQ ID NO. 34 | hairpin | PO₄ - GACGCGATTTTTTCGCGTCATAGTCCCTGGCGTGCTT |
| CO2Pro5 SEQ ID NO. 35 | carboxyl primer | CO₂ - TGACACCGTACCTGCTCT |
| PO4Pro3 SEQ ID NO. 36 | phosphorylated primer | PO₄ - ATAGTCCCTGGCGTGCTT |

| | | |
|---|---|---|
| CO₂: 5' carboxyl group; PO₄: 5' phosphate group | | |

### Separation

The ligated library is separated by magnetic beads conjugated with complementary capture oligonucleotides matching the adapter sequences. The specific hybridization step is performed by a IKingfisher Flex magnetic particle processor (Thermo Fisher Scientific) with each of tl-ie beads remaining bound to a fixed position by the respective magnet. The beads are then transferred to separate microtiter wells for further processing.

### Synthesis

The sorted library is activated by incubation with 20mM DMT-MM, 100mM MOPS pH 6.4 for at least 1 hour at room temperature. The beads with the activated library are subsequently transferred into a new microtiter plate, each of the 16 wells containing a different amino acid and incubated for another 1 hour at room temperature for coupling. The beads with reacted library are finally washed and pooled into one Eppendorf tube.

### Cleavage

The elution step is performed in 450µl distilled water at 94°C and beads are removed quickly. The remaining library is supplemented with 50µl of lOx NEI33 buffer and incubated for 1 hour at 22°C with 500u endonuclease 1V.
The processed DNA is purified and five further decoding steps are performed as described above until the hProLib6x2N is fully synthesized.

The Selection of the resulting peptide library hProLib6x2N is performed by a binding step to Ni-NTA coupled magnetic beads. Several washing steps can be performed to remove unbound library from the magnetic beads. A final elution step is performed with 5mM EDTA. The eluted library is purified and amplified by PCR using CO2Pro5 and P04Pro3 primers. The dsDNA PCR product is converted to ssDNA by digestion with lambda exonuclease. The enriched ssDNA library is ligated to a phosphorylated hairpin oligonucleotide P04hpPro3 to be used as a template for another two synthesis and selection cycles as described above.
The DNA of the three selection cycles is sequenced by SOLEXA in order to identify enriched sequences that can be translated to peptide sequences specifically binding to Ni-NTA.

## Claims

1. A method for synthesizing a combinatorial library comprising the following steps:
(i) providing a library of template nucleic acids that are partially double-stranded, wherein each template nucleic acid comprises a reactive group, and wherein the two strands in the double-stranded region are covalently linked,
(ii) contacting said template nucleic acids with a library of adapter oligonucleotides, wherein each adapter oligonucleotide of the library comprises a first sequence of 2 or more defined nucleotide residues at one end, and a second sequence of from 7 to 60 nucleotide residues,
(iii) ligating those of the adapter oligonucleotides to the last nucleotide of the double-stranded portion of the template nucleic acid which have hybridized with their first sequence to the single stranded portion of the template nucleic acid in immediate proximity to the double-stranded portion of the template nucleic acid,
(iv) sorting said template nucleic acids according to the second sequences of the adapter oligonucleotides,
(v) chemically modifying said reactive group of the template nucleic acids,
(vi) removing the second sequence of the adapter oligonucleotide by cleavage of the ligated adapter oligonucleotides between the first sequence and the second sequence or by enzymatic digestion with an exonuclease,
(vii) repeating steps (ii) to (vi) as required.

2. The method according to claim 1, wherein the adapter oligonucleotide additionally comprises a linker sequence of 1 to 10 random or degenerated residues in the second sequence adjacent to the first sequence.

3. The method according to claim 1 or 2, wherein the libraries are libraries of compounds selected from the group consisting of organic compounds, peptides, polypeptides, oligonucleotides or oligosaccharides.

4. The method according to any of the preceding claims, wherein in step (iv) the template nucleic acids are sorted into defined reaction compartments, preferably on a chip or in microtiter wells.

5. The method according to any of the preceding claims, wherein the adapter oligonucleotide is attached with the end distal from the first sequence to a solid phase.

6. The method according to any of the preceding claims, wherein sorting in step (iv) is by hybridizing an oligonucleotide probe to the second sequence.

7. The method according to any of the preceding claims, wherein in step (vii) the template nucleic acids are pooled in one compartment.

8. The method according to any of the preceding claims, wherein the linker sequence of the adapter oligonucleotide comprises an abasic site at the position proximate to said first sequence.

9. The method according to any of the preceding claims, wherein the first sequence of the adapter oligonucleotide comprises an LNA nucleotide at the next but one position to the linker sequence.

10. The method according to any of the preceding claims, wherein the linker sequence is RNA or comprises ribonucleotide residues in immediate proximity to the first sequence of the adapter oligonucleotide.

11. The method according to any of the preceding claims, wherein the capture oligonucleotide probes are attached to the reaction compartments.

12. The method according to any of the preceding claims, wherein the capture oligonucleotide probes are attached to beads, preferably magnetic beads, in the reaction compartments.

13. A kit for the synthesis of a combinatorial library comprising in one or more containers:
(i) a library of template nucleic acids that are partially double-stranded, wherein each template nucleic acid comprises a reactive group and wherein the two strands in the double-stranded region are covalently linked;
(ii) a library of adapter oligonucleotides, wherein each adapter oligonucleotide of the library comprises a first sequence of 2 or more defined nucleotide residues at one end, and a second sequence of from 7 to 60, nucleotide residues;
(iii) optionally a library of capture oligonucleotide probes having sequences hybridizing under stringent conditions to the second sequences of the adapter oligonucleotides;
(iv) optionally reagents for modifying the reactive groups of the template nucleic acids;
(v) optionally one or more aqueous buffer solutions, e.g. a stripping buffer solution to remove probes after hybridisation and detection,
(vi) optionally an endonuclease, preferably Endonuclease IV, and
(vii) optionally a ligase, preferably T4 DNA ligase.

14. A method for the selection of compounds for a desired property, comprising the steps of:
(I) synthesizing a combinatorial library with a method according to any one of the claims 1 to 12,
(II) determining those members of the library which have the desired property.

15. The method according to claim 14, wherein the desired property is binding to a target molecule with at least a desired affinity and wherein the method comprises the step of contacting the members of the library with the target molecule.
